# FASCICULE DE BREVET EUROPEEN

(11) **EP 2 504 026 B1**
(45) Date de publication et mention de la délivrance du brevet: **08.04.2015**
(21) Numéro de dépôt: 11778574.1
(22) Date de dépôt: 28.10.2011
(51) Int. Cl.: A61K 38/48, A61K 38/46, A61P 3/04

(54) **COMPOSITION COMPRENANT EN ASSOCIATION AU MOINS UNE ENZYME PROTÉOLYTIQUE ET AU MOINS UNE ENZYME LIPOLYTIQUE POUR SON UTILISATION POUR EMPÊCHER LA SYNTHÈSE DES TRIGLYCÉRIDES**
ZUSAMMENSETZUNG MIT EINER KOMBINATION AUS MINDESTENS EINEM PROTEOLYTISCHEN ENZYM UND MINDESTENS EINEM LIPOLYTISCHEN ENZYM ZUR VERWENDUNG BEI DER SYNTHESE VON TRIGLYCERIDEN
COMPOSITION COMPRISING A COMBINATION OF AT LEAST ONE PROTEOLYTIC ENZYME AND AT LEAST ONE LIPOLYTIC ENZYME, FOR USE IN PREVENTING TRIGLYCERIDE SYNTHESIS

(30) Priorité: 29.10.2010 FR 1058957
(43) Date de publication de la demande: 03.10.2012
(73) Titulaire: Imarko Research S.A., 2227 Luxembourg (LU)
(72) Inventeur: ROMBI, Max, I-18022 Bordighera (IT)
(74) Mandataire: Regimbeau
(86) Numéro de dépôt international: PCT/EP2011/069045
(87) Numéro de publication internationale: WO 2012/056024

(56) Documents cités:
- EP-A1- 1 459 738
- WO-A1-2006/044529
- WO-A1-2009/120760
- WO-A1-2010/025126
- WO-A1-2010/080830
- WO-A1-2010/080835
- WO-A2-03/066088
- WO-A2-2006/136160
- WO-A2-2007/047205
- WO-A2-2007/100675
- WO-A2-2008/019417
- WO-A2-2008/045148
- GB-A- 2 396 297
- US-A- 5 223 425
- US-A- 6 011 001
- US-A1- 2003 095 961
- US-A1- 2005 059 567

## Description

La présente invention concerne composition comprenant en association au moins une enzyme protéolytique choisie parmi la subtilisine ou la nagarse, et au moins une enzyme lipolytique choisie parmi une lipase Cal A ou Cal B de candida anthartica, geotrichum candidum, candida rugosa, ou un mélange de ces lipases, pour son utilisation dans la prévention ou le traitement de l'obésité en empêchant la synthèse des triglycérides, en dégradant le 2-monoacylglycérol dans l'intestin. L'invention a également pour objet une utilisation non thérapeutique d'une composition comprenant en association au moins une enzyme protéolytique choisie parmi la subtilisine ou la nagarse, et au moins une enzyme lipolytique choisie parmi une lipase Cal A ou Cal B de candida anthartica, geotrichum candidum, candida rugosa, ou un mélange de ces lipases, pour prévenir ou diminuer les surcharges pondérales, en empêchant la synthèse des triglycérides, en dégradant le 2-monoacylglycérol dans l'intestin. L'invention concerne également un produit contenant:
- au moins une enzyme protéolytique, choisie parmi la subtilisine ou la nagarse, et
- au moins une lipase choisie parmi une lipase Cal A ou Cal B de candida anthartica, geotrichum candidum, une lipase candida rugosa, ou un mélange de ces lipases,

et l'utilisation non thérapeutique d'un produit contenant:
- au moins une enzyme protéolytique, choisie parmi la subtilisine ou la nagarse, et
- au moins une lipase choisie parmi une lipase Cal A ou Cal B de candida anthartica, geotrichum candidum, une lipase candida rugosa, ou un mélange de ces lipases,
comme produit de combinaison pour une utilisation simultanée, séparée ou étalée dans le temps, pour prévenir ou diminuer les surcharges pondérales, en empêchant la synthèse des triglycérides, en dégradant le 2-monoacylglycérol dans l'intestin.

De manière générale, on admet aujourd'hui que l'augmentation de la fréquence de surcharge pondérale et de l'obésité, du diabète de type II, des maladies cardiovasculaires et le manque d'énergie chez de nombreuses personnes dans les pays développés est due à une mauvaise utilisation des calories contenues dans les produits que nous consommons qui sont la plupart du temps très riches en corps gras. Cette déficience métabolique ou syndrome métabolique affecte près de 50 millions d'américains (presque un américain adulte sur 4) et environ 30 millions d'européens. Environ 7% des adultes entre 20 et 30 ans et 40% des adultes de plus de 40 ans présentent les critères pour développer ce syndrome.

Le syndrome métabolique représente un ensemble de facteurs qui surviennent en même temps et augmentent le risque de maladies cardio-vasculaires, d'attaques cérébrales et de diabète de type II. Le fait de présenter uniquement un seul de ces facteurs - augmentation de la tension artérielle, taux élevé d'insuline, excès de graisse autour de la taille ou taux anormal de cholestérol - favorise le risque de contracter une maladie grave et dans le cas de plusieurs facteurs réunis, le risque est encore plus élevé.

Les corps gras, de par leurs propriétés physiques, chimiques et physiologiques, jouent un rôle important dans la nutrition de l'homme. En plus des matières grasses ingérées par l'homme sous forme d'aliment, l'organisme fabrique lui-même des lipides.

Les corps gras alimentaires, et ceux des tissus adipeux, sont constitués surtout de triglycérides. Un triglycéride est une molécule de glycérol comportant 3 acides gras fixés sur cette molécule. Les triglycérides sont classés dans les lipides (corps gras), mais seuls les 3 acides gras sont des corps gras, le glycérol étant un polyol qui appartient plutôt à la classe des glucides. Par exemple, le glycérol est soluble dans l'eau et pas dans l'huile ; le glycérol n'est donc pas un corps gras.

C'est la différence de structure des acides gras qui sont fixés sur le glycérol qui fait que ces corps gras sont différents et qui influe sur l'état de viscosité de ces corps gras. Il existe ainsi une grande diversité de triglycérides dans les aliments tels que dans les huiles alimentaires d'olive, de tournesol, de soja, le beurre, le suif, le saindoux, les margarines, etc.

Ces acides gras sont assimilés dans l'intestin selon un processus commun à tous les triglycérides qui ne sont pas eux-mêmes assimilables.

Ils doivent être décomposés en diglycérides, monoglycérides, et acides gras libres, grâce à l'action de lipases au niveau du tube digestif, et seuls ces différents produits sont assimilables, les triglycérides ne l'étant pas.

Les lipases ou enzymes lipolytiques sont des enzymes que nous secrétons au niveau de la bouche, de l'estomac et du pancréas qui découpent les triglycérides (TAG) en molécules plus petites pour être assimilables. L'attaque des triglycérides (TAG) par les lipases se fait malheureusement seulement en positions 1 et 3 du glycérol, conduisant à la formation d'acides gras libres et de 2-monoacylglycérol (molécule de glycérol comportant 1 acide gras fixé en position 2 sur cette molécule).

Si l'action des lipases est encore plus importante, on peut même aller jusqu'à obtenir des acides gras libres et du glycérol. Toutes ces molécules sont assimilables par l'organisme.

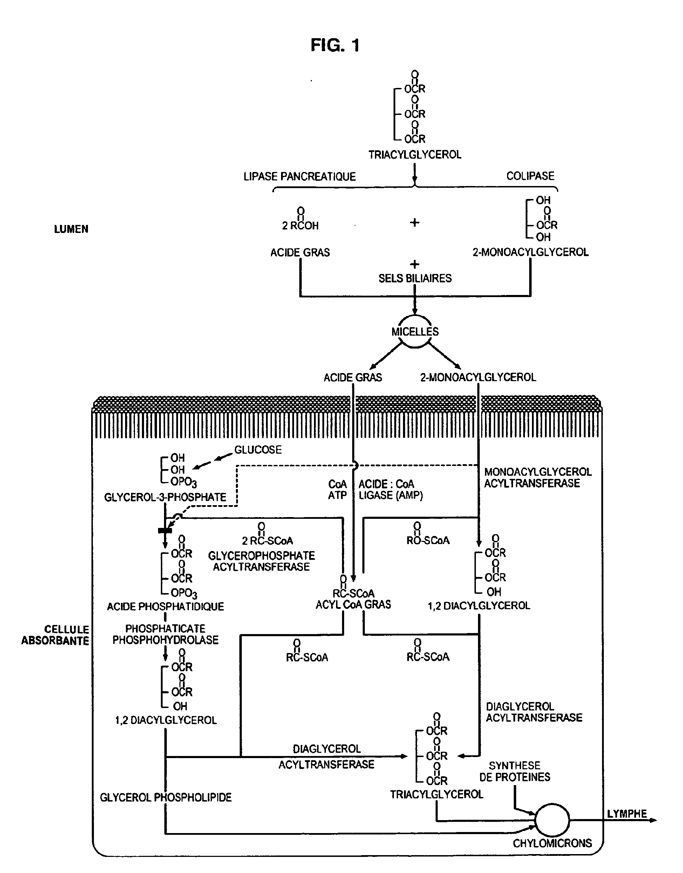

Mais c'est là que les choses se compliquent : dès que ces produits sont assimilés, l'organisme re-synthétise (reforme) dans les cellules épithéliales de l'intestin - les entérocytes - les triglycérides qui avaient été décomposés notamment en acides gras libres et en 2-monoacylglycérol par les lipases telles que les lipases pancréatiques (Cf. Figure 1 qui représente la décomposition des triglycérides (triacylglycérol TAG) par les lipases, puis la reformation des triglycérides dans les cellules de l'intestin).

Cette re-formation des triglycérides (TAG) se fait en particulier grâce à l'activité de 4 enzymes (l'Acyl-coA synthétase, la Monoglycéride transacylase, la Diglycéride transacylase et la Diglycéride synthétase) qui vont agir en plusieurs étapes de la synthèse.

Les acides gras libres vont alors être refixés sur le 2-monoacylglycérol en positions 1 et 3 et reformer des triglycérides. Ces triglycérides, qui ne sont pas solubles dans le sang, vont devoir intégrer des chylomicrons qui vont évoluer vers les VLDL, LDL et HDL et transporter dans tout l'organisme les triglycérides (TAG), le cholestérol et les phospholipides.

Ces nouveaux triglycérides vont ainsi se fixer dans les lipoprotéines et vont être stockés dans les adipocytes (cellules graisseuses). Si bien que les triglycérides qui ont été assimilés par l'organisme, après avoir été décomposés en corps gras plus petits, sont stockés de nouveau comme triglycérides. Ainsi, l'organisme n'utilise pas leur énergie immédiatement puisqu'ils sont stockés dans les adipocytes. L'organisme ne peut pas métaboliser les acides gras lorsqu'ils sont fixés sur du glycérol, comme dans les triglycérides. Pour être métabolisés, les acides gras doivent être libres.

Cette déficience métabolique, que certains appellent le gène de l'obésité (James V. NEEL - Diabetes Mellitus: a « Thrifty » genotype rendered detrimental by « progress » - American Journal of Human Genetics - Vol. 14 - pages 353-362), que constituent la recomposition et le stockage des triglycérides, a été un avantage aux temps des premiers hommes, il y a des milliers d'années. En effet, cette faculté de stocker les corps gras était essentielle à la survie. Durant les périodes fastes, lorsque la chasse permettait de tuer du gibier, les hommes mangeaient beaucoup et mettaient les graisses en réserve. Les hommes en stockant les graisses de la sorte pouvaient ensuite supporter de longues périodes de famine et consommaient leurs réserves de graisse pendant ces dures périodes.

Tout ceci a été bien expliqué par Stephen Cunnane dans son livre « The survival of the fattest », (The Key tu Human Brain Evolution, World Scientific Publishing Co, New Jersey, États-Unis, 2005, www.worldscibooks.com) qui explique que ce sont les personnes avec les réserves de graisses les plus importantes qui ont le mieux survécu aux grandes périodes de disettes.

Ce sont ces mêmes personnes qui ont enrichi leur cerveau en acides gras de la série oméga-3 et qui sont devenues plus intelligentes et plus évoluées que leurs congénères. A cette époque, la déficience métabolique était donc un avantage évolutif, et était aussi un avantage face à la sélection naturelle. C'est en particulier comme cela qu'a été créé l'homo sapiens à partir d'homos primitifs.

L'exemple des Mélanésiens est flagrant. Ce peuple a quitté Taïwan sur des pirogues à balancier puis a colonisé toutes les îles du Pacifique. Ceux qui ont résisté au voyage sont ceux qui avaient la plus grande réserve de graisses.

La sélection naturelle a favorisé la survie des plus gros, qui ont ensuite eu des enfants avec le gène de l'obésité. C'est ainsi que 60 à 70% des Mélanésiens sont diabétiques de type II.

Mais, de nos jours, dans les pays développés où l'on mange à notre faim voire même en excès, cette faculté de stockage devient une catastrophe. En effet, la resynthèse des triglycérides dans les entérocytes ou cellules de l'intestin a des conséquences désastreuses pour la santé des êtres humains. En particulier, cette re-formation des triglycérides induit :
- une augmentation de la taille et du nombre de chylomicrons qui sont les transporteurs des triglycérides, du cholestérol et des phospholipides dans le sang,
- une augmentation des risques d'athérosclérose, facilitant la formation de plaques dans les artères et souvent la thrombose des artères, à cause de tous les acides gras piégés dans les triglycérides, non métabolisés par l'organisme,
- une élévation du taux de mauvais cholestérol en circulation,
- une augmentation en nombre et en taille des cellules graisseuses et donc un développement de la prise de poids ou de l'obésité.

En outre, l'excès de triglycérides dans le sang réduit la quantité d'acides gras libres disponibles pour être métabolisés ; ainsi, nous manquons d'énergie.

Donc dans certains cas graves, où le gène de l'obésité est très marqué, même après un repas, nous avons le sentiment de ne pas avoir de nutriments disponibles dans notre sang. Ce sentiment désagréable fait que nous avons envie de grignoter, que nous avons toujours une sensation de faim qui nous invite à manger et boire pour nous porter mieux.

Cette déficience métabolique, le fait de ne pas pouvoir utiliser les calories contenues par les acides gras que nous assimilons, mais qui sous forme de triglycérides ne sont pas utilisables, car piégés dans les triglycérides, entraîne un ensemble de complications, appelées syndrome X par Reaven ou « deadly quartet » par Kaplan (- G.M. Reaven - Stanford University School of Medicine - division of Endocrinology, Gerontology and Metabolism, Department of Vétérans Affairs Médical Center. Palo Alto. CA. USA - Syndrom X : 6 years later - Journal of Internal Medicine 1994; 236 (Supplement 736): 13-22;
- G.M. Reaven - Simon & Schuster - Rockefeller Center, 1230 Avenue of the Americas - New York , NY 10020 - Syndrome X - Overcoming the silent killer that can give you a heart attack - Copyright 2000 G.M. Raven, T. Strom et B. Fox;
- Norman M. Kaplan MD - The Deadly Quartet - Arch Inter. Med - vol 149, July 1989: 1514-1520).

Pour retrouver l'énergie dont il a besoin, notre organisme, privé d'acides gras disponibles pour êtres métabolisés, va utiliser le glucose, omniprésent dans l'organisme. C'est ce qui nous fait dire que nous sommes un véhicule hybride. Mais le glucose, à l'opposé des acides gras, a de nombreux inconvénients :
- En brûlant, il ne dégage que 4 calories au gramme, alors que les acides gras dégagent 9 calories au gramme,
- Mais surtout, le glucose, pour être utilisé et pénétrer dans les cellules, a besoin de l'hormone insuline. Aussi, pour que le glucose pénètre dans toutes nos cellules, l'organisme doit sécréter encore plus d'insuline, au-delà de ses capacités, et dans des conditions extrêmes. Si bien qu'à un moment, notre organisme et en particulier le pancréas ne peut plus sécréter tant d'insuline et nous avons le phénomène de la résistance à l'insuline ; le glucose s'accumule dans le sang et ne peut plus pénétrer dans les cellules. On appelle cela l'intolérance au glucose ; l'organisme devient insulino-résistant.

Cette hyper insulinémie a des effets pervers ; trop d'insuline arrête la sortie des acides gras des cellules graisseuses, ce que l'on appelle la lipolyse. Il y a donc encore davantage de carence en acides gras disponibles pour être utilisés, il n'y a plus de sorties des acides gras des cellules graisseuses, donc pas de possibilité de perdre du poids notamment pour ceux qui sont atteints d'obésité. Ainsi, la carence en acides gras disponibles est encore aggravée, et on constate une utilisation encore plus forte du glucose dans le métabolisme. D'où, se produisent encore d'autres complications, comme une augmentation de la triglycéridémie et de la cholestérolémie.

Tous ces troubles métaboliques entraînent une dyslipidémie, de l'hypertension, de l'hyper uricémie et un taux de glucose au-dessus de la normale, car le glucose pénètre mal dans les cellules et s'accumule dans le sang provoquant une hyper glycémie.

Toutes ces complications métaboliques peuvent ensuite entraîner le diabète de type 2, qui va s'installer durablement. Une accumulation de corps gras dans les cellules graisseuses, en particulier dans le ventre et autour de la ceinture pour les hommes et dans les fesses pour les femmes, va s'installer également durablement.

Tous ces troubles métaboliques vont avoir des retentissements sur le moral de l'individu qui est alors souvent au plus bas pouvant jusqu'à entraîner un déséquilibre moral ou une dépression nerveuse chez ledit individu souffrant de ces troubles métaboliques, avec notamment un grand sentiment d'insatisfaction et une faim constante.

En outre, le cerveau est alors mal irrigué. L'excès d'insuline déséquilibre tous les paramètres sanguins qui sont perturbés : les adipocytokines, TNPβ, IL-6, PAI 1, adiposine, angiotensinogène, leptine, adiponectine, résistine, MCSF, TGFL, etc... sont en augmentation dans le sang. Tous ces produits inflammatoires accentuent les déséquilibres biologiques et déstabilisent le métabolisme lipidique et glucidique de l'individu.

Des travaux antérieurs ont cherché de nouvelles manières de court-circuiter la synthèse de triglycérides et de pallier cette déficience métabolique.

Ainsi, différentes méthodes ont été trouvées pour déstocker les cellules graisseuses et avoir la biodisponibilité des acides gras stockés dans ces cellules graisseuses.

On a pour cela notamment utilisé des substances, telles que l'extrait de thé riche en flavonoïdes pour bloquer l'enzyme O'-methyl transférase qui détruit l'adrénaline, ou encore l'extrait de pépins de raisin riche en flavanes 3-ol et l'écorce de pin riche en bioflavonoïdes, ces derniers augmentant tous deux la thermogénèse, c'est-à-dire qu'ils libèrent les acides gras des cellules graisseuses ; de même, les polyphénols extraits du cacao qui ont le même effet.

C'est pour faire sortir les corps gras prisonniers des cellules graisseuses que l'on a fait un usage très important des amphétamines et autres isomérides ou Mediator. Mais ces produits étaient très dangereux et sont maintenant tous interdits.

On a encore essayé de court-circuiter les cellules graisseuses avec le CLA, acide linoléique conjugué, qui ne rentre pas dans le cycle de la synthèse des TAG (Triacylglycérol), ainsi qu'avec les huiles à chaînes moyennes qui brûlent sans passer par les cellules graisseuses.

La Demanderesse a cherché à trouver un meilleur moyen de s'opposer au syndrome métabolique, combattre le gène de l'obésité et d'utiliser l'énergie des corps gras tout en ne favorisant pas la formation de dépôts graisseux.

La Demanderesse a constaté que de nombreux laboratoires proposaient d'utiliser des agents bloqueurs de lipases afin d'empêcher la décomposition des TAG, ainsi que leur reformation.

Mais, la Demanderesse a découvert de manière surprenante que les produits anti-lipases n'étaient absolument pas efficaces pour perdre du poids, mais qu'ils avaient en réalité une action néfaste sur l'organisme en augmentant la quantité de TAG restant dans l'intestin.

La solution proposée par la présente invention est d'inhiber, réduire et/ou d'empêcher la reformation des triglycérides dans les entérocytes ou cellules de l'intestin de l'organisme par l'utilisation d'une association d'enzyme protéolytique ou protéase avec au moins une enzyme lypolytique ou lipase.

En administrant de telles enzymes protéolytiques, mais surtout des lipases, on évite de manière élégante et sans danger pour l'organisme la re-synthèse des triglycérides.

Ainsi, la Demanderesse a découvert que :
- les enzymes protéolytiques perturbent l'activité des 4 enzymes -l'Acyl-coA synthétase, la Monoglycéride transacylase, la Diglycéride transacylase et la Diglycéride synthétase- dans l'entérocyte, qui aident à la resynthèse des triglycérides,
- mais surtout que si l'on fait agir des lipases particulières qui agissent avantageusement en position 2 sur les triglycérides de l'intestin, on réduit la production du 2 mono acylglycérol, qui est le pivot de la resynthèse des triglycérides. Cette voie métabolique est connue pas tous les biochimistes comme étant la voie de synthèse de 80% des triglycérides ; l'autre voie, moins efficace, étant la resynthèse à partir du glycérol 3 phosphate qui ne produit que 20% des triglycérides.

La présente invention a ainsi pour objet une composition comprenant en association au moins une enzyme protéolytique ou protéase et au moins une enzyme lipolytique ou lipase pour son utilisation pour empêcher la synthèse ou la reformation des triglycérides, notamment dans les entérocytes ou cellules de l'intestin, en décomposant le 2-monoacylglycérol.

Selon la présente invention, l'enzyme protéolytique est la subtilisine ou la nagarse.

Selon une caractéristique particulière de la présente invention, l'enzyme protéolytique est présente à une teneur comprise entre 5 et 30 %, typiquement entre 10 et 20% en poids, par rapport au poids total de la composition.

Selon une autre caractéristique particulière de la présente invention, l'enzyme lipolytique est présente à une teneur comprise entre 70 et 95 %, typiquement entre 80 et 90% en poids, par rapport au poids total de la composition.

Avantageusement selon la présente invention, la composition permet l'administration d'une dose journalière d'enzyme protéolytique comprise entre 10 et 200 mg, plus particulièrement entre 10 et 100 mg, typiquement entre 20 et 100 mg, par exemple entre 50 et 100 mg.

Avantageusement selon la présente invention, la composition permet l'administration d'une dose journalière d'enzyme lipolytique comprise entre 100 et 400 mg, plus particulièrement entre 100 et 300 mg, typiquement entre 100 et 200 mg ou entre 200 et 300 mg.

Dans un mode de réalisation particulier de la présente invention, la composition est formulée pour être administrée par voie orale.

De manière avantageuse selon la présente invention, la composition se présente sous forme de gélule, capsule, comprimé, granule, poudre ou solution buvable.

Selon une caractéristique particulière de la présente invention, la composition se présente sous forme de gélule ou de comprimé gastro-résistant.

Avantageusement, la composition est gastro-résistante afin que la composition libère l'enzyme protéolytique et l'enzyme lipolytique au niveau de l'intestin.

De manière avantageuse selon la présente invention, la composition est une composition pharmaceutique, cosmétique, nutraceutique ou alimentaire, un complément alimentaire, ou encore un dispositif médical, et peut comprendre tout véhicule ou excipient approprié, acceptable du point de vue pharmaceutique, cosmétique, alimentaire ou nutraceutique, ainsi que des additifs conventionnels, connus de l'homme du métier.

Les lipases utilisées dans le cadre de la présente invention sont actives en position 2 sur les triglycérides et vont réduire le 2 monoacylglycérol et empêcher la reformation des triglycérides, voie qui produit 80% des triglycérides.

Selon l'invention, la composition comprend au moins une lipase choisie dans le groupe constitué par la lipase Cal A ou Cal B de candida anthartica, geotrichum candidum, candida rugosa, et des mélanges binaires ou ternaires de ces lipases. Typiquement, la composition comprend un mélange de Cal A ou Cal B de candida anthartica, geotrichum candidum et de candida rugosa.

La composition selon la présente invention est destinée à être utilisée dans la prévention ou la diminution des surcharges pondérales, typiquement dans une composition cosmétique ou un dispositif médical.

La composition ou le produit selon la présente invention est également destiné à être utilisé dans la prévention ou le traitement de l'obésité.

La présente invention a également pour objet une composition ou un produit contenant:
- au moins une enzyme protéolytique, choisie parmi la subtilisine ou la nagarse, et
- au moins une lipase, choisie parmi une lipase Cal A ou Cal B de candida anthartica, geotrichum candidum, une lipase candida rugosa, ou un mélange binaire ou ternaire de ces lipases,
comme produit de combinaison pour une utilisation simultanée, séparée ou étalée dans le temps, pour empêcher la synthèse des triglycérides, en dégradant le 2-monoacylglycérol dans l'intestin.

Les protéases sont typiquement administrées sous forme de gélule. Les protéases sont avantageusement administrées 2 à 3 fois par jour, 1 gélule à chaque repas.

Typiquement, le produit comprend au moins une lipase de candida anthartica telle que la lipase Cal A ou Cal B de candida anthartica, une lipase de geotrichum candidum, et/ou une lipase de candida rugosa. Ceci est particulièrement avantageux car ces lipases agissent en position 2 sur les triglycérides et viennent ainsi dégrader le 2-monoacylglycérol et aider les protéases à empêcher la reformation des triglycérides dans l'organisme. Sans 2-monoacylglycérol, la resynthèse des TAG est bien plus difficile (n'oublions pas que cette voie produit 80% des TAG dans l'organisme).

Les lipases sont typiquement administrées sous forme de gélule. Les lipases sont avantageusement administrées 3 fois par jour, 1 gélule à chaque repas.

Dans un mode de réalisation particulier, la composition ou le produit selon la présente invention est destinée à être utilisée comme médicament, comme agent cosmétique, comme dispositif médical, comme composition alimentaire, comme nutraceutique ou complément alimentaire.

Dans un mode de réalisation particulier, la composition ou le produit selon la présente invention est destinée à être utilisée dans la prévention ou la diminution des surcharges pondérales.

Dans un autre mode de réalisation particulier, la composition ou le produit selon la présente invention est destinée à être utilisée dans la prévention ou le traitement de l'obésité.

Les exemples suivants sont destinés à illustrer l'invention sans aucunement en limiter la portée.

### Exemple 1 : Etude de l'activité induite par l'administration d'une association d'enzymes protéolytiques, telles que la subtilisine, et d'enzymes lipolytiques, telles que la cal A de candida anthartica, la lipase de geotrichum candidum, et la lipase de candida rugosa, pour empêcher la synthèse des triglycérides :

Une étude d'activité et de dose test a été réalisée sur des rats. Ces rats étaient répartis par 10, en 3 groupes (bras).

Le premier bras, groupe témoin, n'a reçu aucun produit actif.

Les 2 autres bras, groupes de 10 rats chacun, ont reçu pendant 8 jours (une fois par jour) respectivement un mélange de 50 mg de subtilisine et 100 mg de lipase cal A de candida anthartica pour le groupe 1, et un mélange de 50 mg de subtilisine et 50 mg de lipase de geotrichum candidum, ainsi que 50 mg de lipase de candida rugosa pour le groupe 2.

Au bout de 8 jours, tous les rats, y compris le groupe témoin, ont reçu une dose d'huile d'olive, équivalant chez l'homme à une cuillère à soupe (15 ml).

Après cette administration d'huile d'olive à tous les rats, des prises de sang ont été pratiquées sur tous les rats, 1 h après l'administration de l'huile d'olive, 2 h et demi après et 5 h après. La dose de triglycérides a alors été calculée dans le sang prélevé.

On a trouvé chez les rats témoins une dose élevée de TAG dans le sang, pour les 3 prélèvements (1h, 2h et demi et 5h).

Les groupes 1 et 2 n'avaient pas de TAG dans le sang.

L'huile d'olive a ensuite été administrée à nouveau deux fois de plus à une semaine d'intervalle - les bras 1 et 2 continuant tout le temps à recevoir de la subtilisine en association avec les lipases mentionnées ci-dessus.

Les résultats ont été les mêmes ces deux autres fois.

Les groupes 1 et 2 n'ont pas reformé de TAG,
tandis que le groupe témoin avait une dose élevée de TAG dans le sang, pour les 3 prélèvements.

On peut donc en conclure que l'association de la subtilisine et des lipases a empêché la formation des triglycérides (TAG)

### Exemple 2 : Etude sur 10 porcs nains

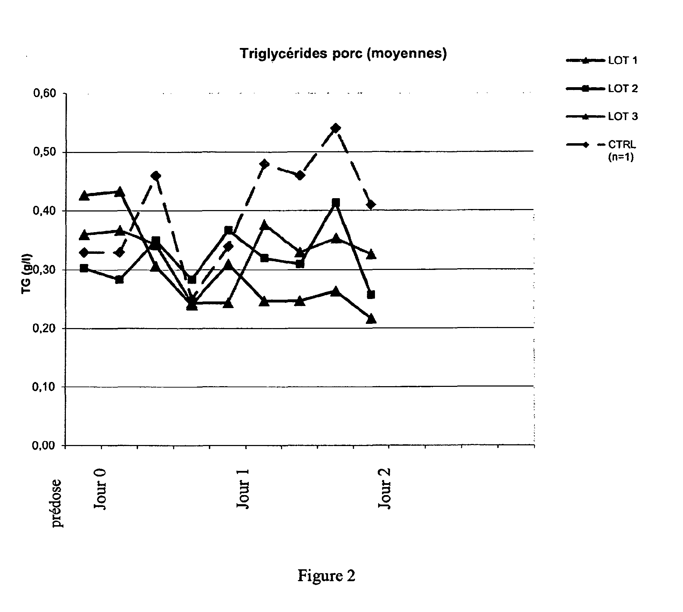

Une étude a été réalisée sur 10 porcs de race naine.
Ces porcs ont été séparés en 4 groupes :
- 1 porc témoin
- 3 porcs lot 1
- 3 porcs lot 2
- 3 porcs lot 3
Le porc témoin n'a pas reçu d'enzyme.
Les 3 autres bras ont reçu trois formules d'enzymes.
La formule 1 était composée de 10 mg de subtilisine et de 300 mg de lipase cal A, ainsi que de la pancréatine. Elle a été administrée aux porcs du lot 1 à un dosage de 20 mg/kg.
La formule 2 était composée de 10 mg de subtilisine et de 300 mg de lipase candida rugosa, ainsi que de la pancréatine. Elle a été administrée aux porcs du lot 2 à un dosage de 40 mg/kg.
La formule 3 était composée de 10 mg de subtilisine et de 300 mg de lipase_geotrichum candidum, ainsi que de la pancréatine. Elle a été administrée aux porcs du lot 3 à un dosage de 40 mg/kg.
Les formules ont été données pendant 8 jours, une fois par jour.
Les résultats (mesure du taux de triglycérides) ont été mesurés par des prises de sang toutes les 2 heures pendant 2 jours.
Les résultats de l'étude ont montré que le porc témoin produisait beaucoup de triglycérides, tandis que les 3 autres bras ne fabriquaient que peu de triglycérides (Cf. Figure 2).
Une seconde étude a été réalisée 8 jours plus tard en prenant cette fois comme porcs témoins 1 porc de chaque bras mentionné ci-dessus (1 porc de lot 1, 1 porc de lot 2, et 1 porc de lot 3).
Les 2 porcs restants par lot ont reçu les mêmes formules d'enzymes que celles mentionnées ci-dessus.
Il a alors été constaté que l'ensemble des porcs ne fabriquaient pas beaucoup de triglycérides, y compris les porcs témoins. On peut ainsi en conclure que les enzymes données 8 jours plus tôt avaient encore une activité sanguine avec un effet retard, et que les lipoprotéines ont été réduites sur les animaux témoins qui avaient reçu les enzymes 8 jours plus tôt.

## Revendications

1. Composition comprenant en association au moins une enzyme protéolytique choisie parmi la subtilisine ou la nagarse, et au moins une enzyme lipolytique choisie parmi une lipase Cal A ou Cal B de candida anthartica, geotrichum candidum, candida rugosa, ou un mélange de ces lipases, pour son utilisation dans la prévention ou le traitement de l'obésité en empêchant la synthèse des triglycérides, en dégradant le 2-monoacylglycérol dans l'intestin.

2. Utilisation non thérapeutique d'une composition comprenant en association au moins une enzyme protéolytique choisie parmi la subtilisine ou la nagarse, et au moins une enzyme lipolytique choisie parmi une lipase Cal A ou Cal B de candida anthartica, geotrichum candidum, candida rugosa, ou un mélange de ces lipases, pour prévenir ou diminuer les surcharges pondérales, en empêchant la synthèse des triglycérides, en dégradant le 2-monoacylglycérol dans l'intestin.

3. Composition pour son utilisation selon la revendication 1 ou utilisation selon la revendication 2, **caractérisée en ce que** la composition est formulée pour être administrée par voie orale.

4. Composition pour son utilisation selon l'une quelconque des revendications 1 et 3 ou utilisation selon l'une quelconque des revendications 2 et 3, **caractérisée en ce que** la composition se présente sous forme de gélule, capsule, comprimé, granule, poudre ou solution buvable.

5. Composition pour son utilisation selon la revendication 4 ou utilisation selon la revendication 4, **caractérisée en ce que** la composition se présente sous forme de gélule ou de comprimé gastro-résistant.

6. Composition pour son utilisation selon l'une quelconque des revendications 1 et 3 à 5 ou utilisation selon l'une quelconque des revendications 2 à 5, **caractérisée en ce que** la composition permet l'administration d'une dose journalière d'enzyme protéolytique comprise entre 50 et 100 mg.

7. Composition pour son utilisation selon l'une quelconque des revendications 1 et 3 à 6 ou utilisation selon l'une quelconque des revendications 2 à 6, **caractérisée en ce que** la composition permet l'administration d'une dose journalière d'enzyme lipolytique comprise entre 200 et 300 mg.

8. Produit contenant:
- au moins une enzyme protéolytique, choisie parmi la subtilisine ou la nagarse, et
- au moins une lipase choisie parmi une lipase Cal A ou Cal B de candida anthartica, geotrichum candidum, une lipase candida rugosa, ou un mélange de ces lipases,
comme produit de combinaison pour une utilisation simultanée, séparée ou étalée dans le temps, dans la prévention ou le traitement de l'obésité, en empêchant la synthèse des triglycérides, en dégradant le 2-monoacylglycérol dans l'intestin.

9. Utilisation non thérapeutique d'un produit contenant:
- au moins une enzyme protéolytique, choisie parmi la subtilisine ou la nagarse, et
- au moins une lipase choisie parmi une lipase Cal A ou Cal B de candida anthartica, geotrichum candidum, une lipase candida rugosa, ou un mélange de ces lipases,
comme produit de combinaison pour une utilisation simultanée, séparée ou étalée dans le temps, pour prévenir ou diminuer les surcharges pondérales, en empêchant la synthèse des triglycérides, en dégradant le 2-monoacylglycérol dans l'intestin.

## Patentansprüche

1. Zusammensetzung enthaltend in Kombination wenigstens ein proteolytisches Enzym gewählt aus Subtilisin oder Nagarse und wenigstens ein lipolytisches Enzym gewählt aus einer Lipase aus Candida antarctica CalA oder CalB, Geotrichum candidum, Candida rugosa oder einer Mischung dieser Lipasen zur Verwendung bei der Prävention oder Behandlung von Fettleibigka, wobei die Triglyceridsynthese durch Degradation von 2-Monoacylglycerol im Dünndarm verhindert wird.

2. Nicht therapeutische Verwendung einer Zusammensetzung, enthaltend in Kombination wenigstens ein proteolytisches Enzym gewählt aus Subtilisin oder Nagarse und wenigstens ein lipolytisches Enzym gewählt aus einer Lipase aus Candida antarctica CalA oder CalB, Geotrichum candidum, Candida rugosa oder einer Mischung dieser Lipasen, zur Prävention oder Reduzierung von Übergewicht, wobei die Triglyceridsynthese durch Degradation von 2-Monoacylglycerol im Darmarm verhindert wird.

3. Zusammensetzung zur Verwendung nach Anspruch 1 oder Verwendung nach Anspruch 2, **dadurch gekennzeichnet, dass** die Zusammensetzung für eine orale Applikation zubereitet ist.

4. Zusammensetzung zur Verwendung nach einem der Ansprüche 1 und 3 oder Verwendung nach einem der Ansprüche 2 und 3, **dadurch gekennzeichnet, dass** die Zusammensetzung die Form von Gelatinekapseln, Kapseln, Tabletten, Granulum, Pulver oder einer trinkbaren Lösung hat.

5. Zusammensetzung zur Verwendung nach Anspruch 4 oder Verwendung nach Anspruch 4, **dadurch gekennzeichnet, dass** die Zusammensetzung die Form von magensaftresistenten Gelatinekapseln oder Tabletten hat.

6. Zusammensetzung zur Verwendung nach einem der Ansprüche 1 und 3 bis 5 oder Verwendung nach einem der Ansprüche 2 bis 5, **dadurch gekennzeichnet, dass** die Zusammensetzung die Applikation einer Tagesdosis von proteolytischen Enzymen zwischen 50 mg und 100 mg erlaubt.

7. Zusammensetzung zur Verwendung nach einem der Ansprüche 1 und 3 bis 6 oder Verwendung nach einem der Ansprüche 2 bis 6, **dadurch gekennzeichnet, dass** die Zusammensetzung die Verabreichung einer Tagesdosis von lipolytischen Enzymen zwischen 200 mg und 300 mg erlaubt.

8. Erzeugnis enthaltend:
- wenigstens ein proteolytisches Enzym gewählt aus Subtilisin oder Nagarse, und
- wenigstens eine Lipase gewählt aus einer Lipase aus Candida antarctica CalA oder CalB, Geotrichum candidum, einer Lipase aus Candida rugosa oder einer Mischung dieser Lipasen,
als Kombinationserzeugnis zur gleichzeitigen, getrennten oder zeitlich versetzten Verwendung bei der Prävention oder Behandlung von Fettleibigkent, wobei die Trig-lyceridsynthese durch Degradation von 2-Monoacylglycerol im Darm verhindert wird.

9. Nicht therapeutische Verwendung eines Erzeugnisses enthaltend:
- wenigstens ein proteolytisches Enzym gewählt aus Subtilisin oder Nagarse, und
- wenigstens eine Lipase gewählt aus einer Lipase aus Candida antarctica CalA oder CalB, Geotrichum candidum, einer Lipase aus Candida rugosa oder einer Mischung dieser Lipasen,
als Kombinationserzeugnis zur gleichzeitigen, getrennten oder zeitlich versetzten Verwendung bei der Prävention oder Reduzierung von Fettleibigkeit, wobei die Triglyceridsynthese durch Degradation von 2-Monoacylglycerol im Darm verhindert wird.

## Claims

1. Composition comprising in association at least one proteolytic enzyme chosen from subtilisin or nagarse, and at least one lipolytic enzyme chosen from a Cal A or Cal B lipase from candida antartica, geotrichum candidum, candida rugosa, or a mixture of these lipases, for use in the prevention or treatment of obesity by preventing triglyceride synthesis, by degrading 2-monoacylglycerol in the intestine.

2. Non-therapeutic use of a composition comprising in association at least one proteolytic enzyme chosen from subtilisin or nagarse, and at least one lipolytic enzyme chosen from a Cal A or Cal B lipase from candida antartica, geotrichum candidum, candida rugosa, or a mixture of these lipases, for preventing or reducing overweight, by preventing triglyceride synthesis, by degrading 2-monoacylglycerol in the intestine.

3. Composition for use according to claim 1 or use according to claim 2, **characterised in that** the composition is formulated to be administered orally.

4. Composition for use according to any of claims 1 and 3 or use according to any of claims 2 and 3, **characterised in that** the composition is presented as a soft capsule, a hard capsule, a tablet, a granule, a powder or an oral solution.

5. Composition for use according to claim 4 or use according to claim 4, **characterised in that** the composition is presented as a gastro-resistant soft capsule or tablet.

6. Composition for use according to any of claims 1 and 3 to 5 or use according to any of claims 2 to 5, **characterised in that** the composition is suitable for administering a daily dose of proteolytic enzyme between 50 and 100 mg.

7. Composition for use according to any of claims 1 and 3 to 6 or use according to any of claims 2 to 6, **characterised in that** the composition is suitable for administering a daily dose of lipolytic enzyme between 200 and 300 mg.

8. Product containing:
- at least one proteolytic enzyme chosen from subtilisin or nagarse, and
- at least one lipase chosen from a Cal A or Cal B lipase from candida antartica, geotrichum candidum, a candida rugosa lipase, or a mixture of these lipases,
as a combination product for simultaneous, separate or staggered use, in the prevention or treatment of obesity by preventing triglyceride synthesis, by degrading 2-monoacylglycerol in the intestine.

9. Non-therapeutic use of a product containing:
- at least one proteolytic enzyme chosen from subtilisin or nagarse, and
- at least one lipase chosen from a Cal A or Cal B lipase from candida antartica, geotrichum candidum, a candida rugosa lipase, or a mixture of these lipases,
as a combination product for simultaneous, separate or staggered use, for preventing or reducing overweight, by preventing triglyceride synthesis, by degrading 2-monoacylglycerol in the intestine.
